# EUROPÄISCHE PATENTSCHRIFT

(11) **EP 1 071 674 B1**
(45) Veröffentlichungstag und Bekanntmachung des Hinweises auf die Patenterteilung: **11.08.2004**
(21) Anmeldenummer: 98964493.5
(22) Anmeldetag: 08.12.1998
(51) Int. Cl.: C07D 263/58

(54) **VERFAHREN ZUR HERSTELLUNG VON CHLORBENZOXAZOLEN**
METHOD FOR PRODUCING CHLOROBENZOXAZOLENE
PROCEDE DE PRODUCTION DE CHLOROBENZOXAZOLS

(30) Priorität: 16.12.1997 DE 19755904
(43) Veröffentlichungstag der Anmeldung: 31.01.2001
(73) Patentinhaber: Bayer CropScience GmbH, 65929 Frankfurt/Main (DE)
(72) Erfinder: RESSEL, Hans-Joachim, D-65795 Hattersheim (DE); ASLAM, Mohammed, D-65779 Kelkheim (DE); DEMOUTE, Jean-Pierre, D-65779 Kelkheim (DE); SCHLEGEL, Günter, Tokyo 158 (JP); WELTER, Wolfgang, D-65719 Hofheim (DE)
(86) Internationale Anmeldenummer: PCT/EP1998/007969
(87) Internationale Veröffentlichungsnummer: WO 1999/031076

(56) Entgegenhaltungen:
- DE-A- 2 059 725
- DE-A- 3 234 530
- DE-A- 3 406 909

## Beschreibung

Die Erfindung betrifft das technische Gebiet der Verfahren zur Herstellung von Zwischenprodukten, die für Synthesen von Wirkstoffen, beispielsweise Wirkstoffe für Pflanzenschutzmittel oder Arzneimittel, eingesetzt werden können.

Chlorbenzoxazole haben bereits große Bedeutung als Zwischenprodukte für Pflanzenschutz- und Arzneimittelwirkstoffe. Ihre Eigenschaften und Verfahren zu deren Herstellung sind unter anderem beschrieben in DE-A-3207153; EP-A-43573 und GB-A-913910.

Nach Verfahren aus den genannten Druckschriften können Chlorbenzoxazole z.B. aus 2-Mercapto-1,3-benzoxazolen unter Austausch der Mercapto-Gruppe durch Chlor unter Verwendung verschiedener Chlorierungsmitteln hergestellt werden. Als Beiprodukte erhält man Schwefelchloride, die entsorgt werden müssen.

Eine weitere Darstellungsmethode führt über die entsprechend substituierten 1,3-Benzoxazol-2-one, die mit überschüssigem Phosphorpentachlorid in die Chlorbenzoxazole überführt werden (EP-A-572893; EP-A-141053; DE-A-3406909). Beispielsweise wird im Falle der Herstellung von 2,6-Dichlorbenzoxazol dann 6-Chlorbenzoxazol-2-on eingesetzt. Die Wiederaufbereitung des dabei eingesetzten Überschusses von PCl₅ erfordert einen besonderen Aufwand.

Es ist bereits bekannt, daß man die unsubstituierte thioanaloge Verbindung 1,3-Benzthiazol durch direkte Chlorierung in Gegenwart von Chlorierungskatalysatoren in 2-Chlor-benz-1,3-thiazol überführen kann (DE-A-3234530). Diese selektive Monochlorierungsreaktion ist jedoch nicht für das analoge Benzoxazol bekannt; vielmehr zeigt DE-A-2059725, daß hierbei Perchlorierung im Molekül stattfindet, ohne Selektivität bei der Besetzung der möglichen Substitutionsplätze.

Es besteht ein Bedarf nach einem alternativen Verfahren zur Herstellung von Chlorbenzoxazolen, welches Nachteile der obengenannten Verfahren nicht besitzt. Überraschenderweise wurde nun gefunden, daß Chlorbenzoxazole durch direkte Chlorierung aus Benzoxazolen erhalten werden können. Dabei können wahlweise sowohl Monochlorierungen als auch bestimmte Dichlorierungen vorgenommen werden.

Gegenstand der Erfindung ist deshalb ein Verfahren zur Herstellung von Chlorbenzoxazolen der Formel (I), 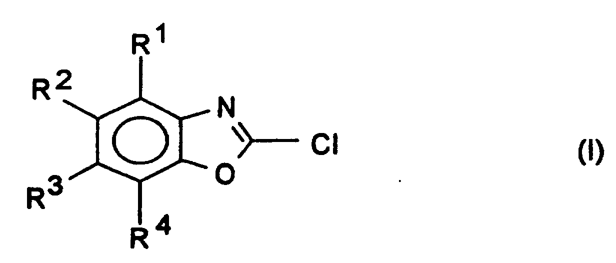 worin R¹, R² und R⁴ jeweils unabhängig voneinander H, Halogen, CN, NO₂, C₁-C₅-Alkyl, C₁-C₅-Alkoxy, Aryl oder Aryloxy, wobei jeder der letztgenannten 4 Reste unsubstituiert oder substituiert ist, bedeuten und
- (Fall a): R³ = H, Halogen, CN, NO₂, C₁-C₅-Alkyl, C₁-C₅-Alkoxy, Aryl oder Aryloxy, wobei jeder der letztgenannten 4 Reste unsubstituiert oder substituiert ist, bedeutet oder
- (Fall b): R³ = Chlor bedeutet,
dadurch gekennzeichnet, daß Benzoxazole der Formel (II), 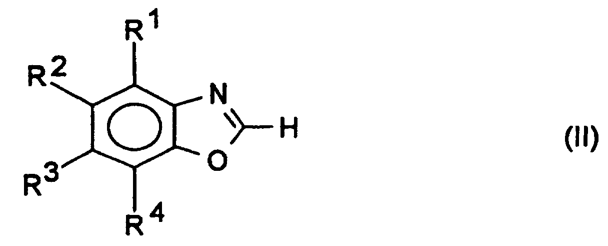 in welcher R¹, R² und R⁴ die gleiche Bedeutung haben wie in Formel (I) und R³ im Fall (a) wie in Formel (I) definiert ist bzw. R³ im Fall (b) Wasserstoff ist,

in Gegenwart eines sauren Katalysators mit einem Chlorierungsmittel zum Monochlorierungsprodukt (I) bzw. im Fall (b) mit einem Überschuß des Chlorierungsmittels zum Dichlorierungsprodukt (I), worin R³ = Chlor bedeutet, umsetzt.

Erfindungsgemäß können die 2-Chlorderivate der Formel (I) selektiv in hoher Ausbeute und Reinheit hergestellt werden. Außerdem zeigen unsere Versuche, daß bei Weiterführung der Chlorierungsreaktion von Benzoxazolen, vorzugsweise unsubstituiertem Benzoxazol, zum entsprechenden 2-Chlorbenzoxazol unter Einsatz von überschüssigem Chlorierungsmittel 2,6-dichlorierte Benzoxazole, vorzugsweise 2,6-Dichlorbenzoxazol, selektiv erhalten werden können. Eine derartige Selektivität war nicht vorauszusehen.

Aufgrund der in DE-A-2059725 beschriebenen Ergebnisse wurde erwartet, daß bei der Chlorierung von Benzoxazol unselektive Mehrfachchlorierung stattfände. Außerdem konnte eine Übertragung der Bedingungen, wie sie für die Chlorierung von Benzthiazol zum 2-Chlorbenzthiazol beschrieben sind (DE-A-3234530), auf das Benzoxazol-Molekül nicht erwartet werden, weil der Benzoxazol-Grundkörper und insbesondere das Benzoxazol selbst als ein viel empfindlicheres (reaktiveres) Molekülsystem bzw. Molekül bekannt ist. Die technischen Lehren aus DE-A-2059725 und DE-A-3234530 waren deshalb wiederspruchsfrei erklärbar. Überraschenderweise lassen sich jedoch unter den erfindungsgemäßen Bedingungen auch mit Benzoxazolen selektive Chlorierungen durchführen, wobei die Chlorderivate der Formel (I) in der Regel in hoher Ausbeute und Selektivität erhalten werden.

Von besonderem Interesse sind erfindungsgemäße Verfahren zur Herstellung von Chlorbenzoxazolen der genannten Formel (I),
worin R¹, R² und R⁴ jeweils unabhängig voneinander H, Halogen, CN, NO₂, C₁-C₅-Alkyl, C₁-C₅-Haloalkyl, C₁-C₅-Alkoxy, C₁-C₅-Haloalkoxy, Phenyl oder Phenoxy, wobei jeder der letztgenannten 2 Reste unsubstituiert oder durch einen oder mehrere Reste aus der Gruppe Halogen, CN, NO₂, C₁-C₄-Alkyl, C₁-C₄-Haloalkyl, C₁-C₄-Alkoxy und C₁-C₄-Haloalkoxy substituiert ist,
vorzugsweise H, Halogen, wie Fluor, Chlor, Brom oder Iod, Methyl, Ethyl, Methoxy, Ethoxy, CF₃, CCl₃, OCF₃ oder OCHF₂,
insbesondere H oder Chlor, bedeuten und
- (Fall a): R³ in Formel (I) einen Rest aus der Gruppe der für R¹, R² und R⁴ möglichen Reste, vorzugsweise H oder Chlor, bedeutet oder
- (Fall b): R³ in Formel (I) Chlor bedeutet.

In den Formeln (I) und (II) können die Reste Alkyl, Alkoxy, Haloalkyl, Haloalkoxy, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgenüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten, z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl.
Halogen bedeutet beispielsweise Fluor, Chlor, Brom oder Iod, Haloalkyl, -alkenyl und-alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andered durch Halogen substituierte Reste.
Aryl bedeutet einen monocyclischen, carbocyclischen aromatischen Ring, der im substituierten Fall auch ein bi- oder polycyclisches aromatisches System einschließt, das mindestens einen aromatischen Ring und gegebenenfalls weitere aromatische Ringe oder teilungesättigte oder gesättigte Ringe enthält; Aryl ist beispielsweise Phenyl, Naphthyl, Tetrahydronaphthyl, Indenyl, Indanyl, Pentalenyl, Fluorenyl und ähnliches, vorzugsweise Phenyl. Aryloxy bedeutet vorzugsweise ein dem genannten Arylrest entsprechender Oxy-Rest, insbesondere Phenoxy.

Substituierte Reste, wie substituiertes Alkyl, Aryl, Phenyl oder Phenoxy bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- oder Dialkylamino, und Alkylsulfinyl, Naloalkylsulfinyl, Alkylsulfonyl, Haloalkylsulfonyl und, im Falle cyclischer Reste, auch Alkyl und Haloalkyl bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Die Ausgangsstoffe, Benzoxazole der Formel (II), können auf bekannte Weise oder analog zu bekannten Verfahren hergestellt werden. Beispielsweise werden durch Reaktion von 2-Aminophenolen mit Orthoameisensäureestem oder mit Ameisensäure oder Formamid Benzoxazole erhalten (Houben-Weyl, "Methoden der organischen Chemie", Bd. E8a).

Geeignete Lösungsmittel für die Chlorierungsreaktion sind unter den Reaktionsbedingungen inerte oder in geeigneter Weise an der Reaktion beteiligte organische oder anorganische Lösungsmittel, wie sie bei Halogenierungsreaktionen üblicherweise eingesetzt werden, oder deren Gemische. In Einzelfällen können auch die Reaktionskomponenten als Lösungsmittel eingesetzt werden.
Beispiele für organische Lösungsmittel sind
- aromatische oder aliphatische Kohlenwasserstoffe wie Benzol, Toluol, Xylol und Paraffine,
- halogenierte aliphatische oder aromatische Kohlenwasserstoffe, z. B. chlorierte Alkane und Alkene, Chlorbenzol, o-Dichlorbenzol,
- Nitrile wie Acetonitril,
- Carbonsäuren und deren Derivate, wie Essigsäure oder deren Ester.
Beispiele für anorganische Lösungsmittel sind
- Phosphoroxychlorid oder SOCl₂, die zugleich auch als Chlorierungsmittel in Frage kommen.

Es kann auch vorteilhaft in Substanz, d. h. in der Schmelze des Ausgangsstoffes (II) oder der Schmelze des Produktes (I) bzw. deren Gemischen gearbeitet werden.

Als Katalysatoren kommen saure Stoffe oder deren Mischungen in Betracht, z.B. Mineralsäuren oder saure Salze davon; saure Ionenaustauscher; Zeolithe (H-Form); andere saure, mineralische Stoffe, wie Montmorillonit oder Lewis Säuren, z.B. Salze von Übergangsmetallen wie FeHal₃, AlHal₃, Sb₂Hal₅, ZnHal₂, SnHal₂, SnHal₄, TiHal₄, CuHal, CuHal₂, etc.; dabei bedeutet Hal jeweils ein Halogen aus der Gruppe Fluor, Chlor, Brom und Iod, vorzugsweise Chlor, Brom oder Iod, insbesondere Chlor. Vorzugsweise werden Eisen-(III)-chlorid, Aluminiumtrichlorid oder Montmorillonit, insbesondere FeCl₃ oder AlCl₃ eingesetzt.

Die Katalysator-Menge kann in weitem Bereich variiert werden. Die optimale Menge an Katalysator hängt vom einzelnen Katalysator ab und beträgt beispielsweise 0,05 bis 10 Molprozent, vorzugsweise 0,1 bis 3 Molprozent Katalysator, bezogen auf die eingesetzte Menge an Verbindung der Formel (II).

Die Temperaturen, bei denen die Reaktionen durchgeführt werden können, können je nach Lösungmittel, den spezifischen Verbindungen der Formel (I) und (II), den Katalysatoren und Chlorierungsmittel in einem weiten Bereich variieren; geeignete Reaktionstemperaturen liegen in der Regel im Bereich von 20 bis 200 °C. Je nachdem, ob eine Monochlorierung oder eine Dichlorierung angestrebt wird oder Mehrfachchlorierungen als Nebenreaktion in Frage kommen, sollte die Reaktionstemperatur zweckmäßig gewählt und gegebenenfalls in Vorversuchen optimiert werden. Vorzugsweise liegt die Temperatur im Bereich von 60 bis 150°C, insbesondere 80 bis 140 °C.

Als ein Chlorierungsmittel kommen im allgemeinen alle für die Chlorierung organischer Verbindungen einsetzbaren Mittel oder deren Mischungen oder Kombinationen in Frage. Geeignete Chlorierungsmittel sind beispielsweise Chlor, SO₂Cl₂, PCl₃, PCl₅, POCl₃, SCl₂, S₂Cl₂, SOCl₂. Es können auch Gemische hieraus oder mit anderen Chlorierungsmitteln verwendet werden. Vorzugsweise wird gasförmiges Chlor eingeleitet oder als Chlorierungsmittel POCl₃, PCl₅ oder SOCl₂ verwendet. Weiter bevorzugt wird eine Kombination aus PCl₃ und Chlor oder PCl₅ und Chlor verwendet, die in situ PCl₅ generiert. Dazu wird beispielsweise PCl₃ bzw. PCl₅ im Unterschuß eingesetzt (dann auch als Co-Chlorierungsmittel bezeichnet), beispielsweise in einer Menge von 0,5 bis 20 Molprozent, vorzugsweise 1-10 Molprozent bezogen auf die Verbindung der Formel (II) eingesetzt, und der Rest an Chlorierungsmittel in Form von Chlorgas zugeführt.
Die Menge an eingesetztem Chlorierungsmittel ist zweckmäßig äquimolar oder in leichtem Überschuß, vorzugsweise von 1,0 bis 1,8 Mol oder auch 1,0 bis 1,2 Mol Chlorierungsmittel pro Mol Verbindung der Formel (II) für den Fall der Monochlorierung (Fall a) oder zweimolar bzw. etwas mehr als zweimolar, vorzugsweise 2,0 bis 2,4 Mol Chlorierungsmittel pro Mol Verbindung der Formel (II) für den Fall der Dichlorierung (Fall b). Die Mengen an Chlorierungsmittel sind entsprechend geringer zu bemessen, wenn das Mittel mehr als ein Moläquivalent Chlor pro Mol des Mittels liefert.

Man verfährt bei der Synthese vorzugsweise so, daß das Edukt (Benzoxazol-Derivat der Formel (II)) in Schmelze oder in Schmelze des Produktes oder in einem geeigneten Lösungsmittel vorgelegt und der Katalysator zugesetzt wird. Gegebenenfalls wird dann Co-Chlorierungsmittel wie PCl₃ oder PCl₅ zugesetzt. Bei der gewünschten Temperatur wird dann unter gutem Rühren langsam Chlor eingeleitet bzw. ein anderes Chlorierungsmittel zudosiert.
Die Durchführung der Reaktion in einem nach dem Gegenstromprinzip arbeitenden Reaktor kann eine wesentlich höhere Umsatzrate bewirken.

Man erhält die gewünschten Produkte selektiv, in guter Reinheit und sehr guter Ausbeute. Hochreine Produkte können z. B. durch Feindestillation erhalten werden.

Die Versuche werden an folgenden Beispielen näher erläutert, ohne daß die Erfindung auf diese Ausführungsformen beschränkt sein soll; Mengenangaben beziehen sich auf das Gewicht, sofern nichts anderes angegeben ist.

### Beispiel 1

In einem Rührkolben mit Gaseinleitungsrohr und Trockeneiskühler wurden 20 g (0,1302 mol) 6-Chlorbenzoxazol und 50 ml Chlorbenzol nach Zusatz von 0,1 g Eisen-III-Chlorid (FeCl₃) auf 100°C erhitzt. Unter gutem Rühren wurden langsam während ca. 4 Stdn. insgesamt 11,0 g (0,155 mol) Chlorgas unter die Flüssigkeitsoberfläche eingeleitet. Der Reaktionsverlauf wurde gaschromatographisch (GC-Analyse) verfolgt. Nachdem das Ausgangsmaterial abreagiert hatte, ließ man den den Ansatz abkühlen.
Gemäß GC-Analyse wurden 95 % des Edukts zum 2,6-Dichlorbenzoxazol umgesetzt. Nach Abziehen des Lösungsmittels konnte das Rohprodukt unter reduziertem Druck destilliert werden. Man erhielt 23,07 g (0,122 mol) 2,6-Dichlorbenzoxazol, GC-Reinheit: 99,5 %, = 93,8 % der Theorie.

### Beispiel 2

In einem Versuch analog Beispiel 1 wurden 11,9 g (0,1 mol) 1,3-Benzoxazol unter gleichen Bedingungen zum 2-Chlorbenzoxazol umgesetzt. Man erhielt 14,35 g 2-Chlorbenzoxazol; GC: 99 %ig = Ausbeute 92,5 % der Theorie.

### Beispiel 3

In einem Versuch analog Beispiel 1 wurden 11,9 g (0,1 mol) Benzoxazol unter Zusatz von 0,5 g Montmorillonit KSF bei 100°C mit Chlorgas umgesetzt.
Das GC zeigte nach Zusatz von 1,1facher molarer Menge Chlorgas einen kompletten Umsatz zum 2-Chlorbenzoxazol. Durch weiteres Einleiten von Chlorgas (zusätzlich 1,0fache molare Menge) bei 120-125°C wurden 80,6 % Umsatz zum 2,6-Dichlorbenzoxazol festgestellt.

### Beispiel 4

10 g (0,065 Mol) 6-Chlorbenzoxazol (>99%ig) wurden in 70 ml Phosphoroxychlorid gelöst und mit 0,26 g trockenem Aluminiumtrichlorid versetzt. Nachdem auf 90 °C erwärmt worden war, wurde unter gutem Rühren Chlorgas unter die Flüssigkeitsoberfläche eingeleitet und der Reaktionsverlauf gaschromatographisch (GC-Analyse) verfolgt. Nach ca. 6 Stunden hatte das Ausgangsmaterial abreagiert. Man kühlte den Ansatz ab und überführte das Reaktionsgemisch in eine Destillationsapparatur mit kurzer Vigreux-Kolonne. In einem Vorlauf wurde das überschüssige POCl₃ abgetrennt. Anschließend destillierte man unter reduziertem Druck eine reine Fraktion 2,6-Dichlorbenzoxazol ab. Man erhielt 11,6 g 2,6-Dichlorbenzoxazol mit einer GC-Reinheit von mehr als 99 %; dies entspricht einer Ausbeute von mehr als 94 % der Theorie.

### Beispiel 5

10 g (0,065 Mol) 6-Chlorbenzoxazol (>99%ig) und 100 ml Chlorbenzol wurden mit 13,54 (0,065) Mol Phosphorpentachlorid und 0,05 g Eisen-III-chlorid (trocken) unter Rühren auf 130-133 °C erhitzt. Nach ca. 6 Stunden war die Reaktion beendet. Man kühlte das Reaktionsgemisch Ansatz ab und filtierte es Ober eine Schicht Kieselgel 60. Nach Eluieren mit Methylenchlorid und Abziehen der Niedrigsieder verbleibt ein in der Kälte erstarrendes Produkt, das nach GC keine weiteren Komponenten enthält; Ausbeute 12,25 g 2,6-Dichlorbenzoxazol (100% d. Th.).

### Beispiel 6

10 g (0,083 Mol) 1,3-Benzoxazol (>99%ig) wurden mit 100 ml POCl₃ und 0,2 g Eisen-III-chlorid (trocken) unter gutem Rühren auf 100 °C erhitzt. Bei dieser Temperatur wurde Chlorgas unter die Flüssigkeitsoberfläche eingeleitet. Die GC-Kontrolle der Reaktion zeigte, daß anfangs 2-Chlorbenzoxazol entstand, das unter weiterer Substitution im Verlauf zu 2,6-Dichlorbenzoxazol abreagierte. Nachdem alles Ausgangsmaterial umgesetzt worden war, wurde die Reaktion abgebrochen. Gemäß GC-Analyse waren 21,5% 2-Chlorbenzoxazol und 71% 2,6-Dichlorbenzoxazol entstanden. Das Rohgemisch wurde destillativ aufgearbeitet. POCl₃ und 2-Chlorbenzoxazol wurden in einer ersten Fraktion gesammelt und konnten direkt für einen weiteren Ansatz eingesetzt werden. Die zweite Fraktion ergab 11,0 g 2,6-Dichiorbenzoxazol (GC >99%ig) (>70% d. Th.). Unter Berücksichtigung der Rückführung des 2-Chlorbenzoxazols ergab sich eine Gesamtausbeute von >92% d. Th.

### Beispiel 7

10 g (0,065 Mol) 6-Chlorbenzoxazol, 0,45 g Phosphortrichlorid und 0,09 g wasserfreies Aluminiumtrichlorid wurden in 30 ml Phosphoroxychlorid (POCl₃) vorgelegt. Unter Erwärmen und Rühren wurde Chlorgas mit einer Rate von 0,6 Äquivatenten Chlor pro Stunde eingeleitet. Nach Erreichen einer Innentemperatur von 80 °C wurde der Chlorgasstrom auf 0,6 Äquivalente Chlor in pro 6 Stunden reduziert und die Temperatur auf 100 °C gesteigert. Die Reaktion wurde gaschromatographisch verfolgt. Nachdem alles Ausgangsmaterial umgesetzt worden war, wurde der Hauptteil des POCl₃ abdestilliert und der Rückstand unter reduziertem Druck einer fraktionierten Destillation unterworfen. Man erhielt eine reine Fraktion von 11,9 g des beim Abkühlen erstarrenden 2,6-Dichlorbenzoxazols (GC >99%ig) (>97% d. Th.).

## Patentansprüche

1. Verfahren zur Herstellung von Chlorbenzoxazolen der Formel (I), 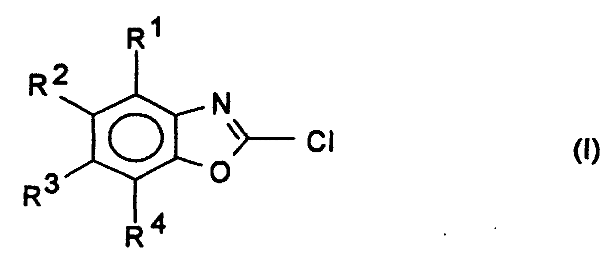 worin R¹, R² und R⁴ jeweils unabhängig voneinander H, Halogen, CN, NO₂, C₁-C₅-Alkyl, C₁-C₅-Alkoxy, Aryl oder Aryloxy, wobei jeder der letztgenannten 4 Reste unsubstituiert oder substituiert ist, bedeuten und
im Fall (a) R³ = H, Halogen, CN, NO₂, C₁-C₅-Alkyl, C₁-C₅ Alkoxy, Aryl oder Aryloxy, wobei jeder der letztgenannten 4 Reste unsubstituiert oder substituiert ist, bedeutet oder
im Fall (b) R³ = Chlor bedeutet,
**dadurch gekennzeichnet, daß** Benzoxazole der Formel (II), 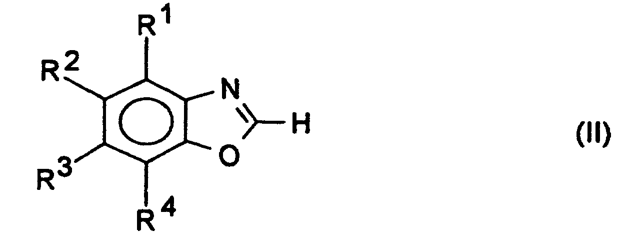 in weicher R¹, R² und R⁴ die gleiche Bedeutung haben wie in Formel (I) und R³ im Fall (a) wie in Formel (I) definiert ist bzw. R³ im Fall (b) Wasserstoff ist,
in Gegenwart eines sauren Katalysators mit einem Chlorierungsmittel zum Monochlorierungsprodukt (I) bzw. im Fall (b) mit einem Überschuß des Chlorierungsmittels zum Dichlorierungsprodukt (I), worin R³ = Chlor bedeutet, umsetzt.

2. Verfahren gemäß Anspruch 1, **dadurch gekennzeichnet, daß**
R¹, R² und R⁴ in Formel (I) jeweils unabhängig voneinander H, Halogen, CN, NO₂, C₁-C₅-Alkyl, C₁-C₅-Haloalkyl, C₁-C₅-Alkoxy, C₁-C₅-Haloalkoxy, Phenyl oder Phenoxy, wobei jeder der letztgenannten 2 Reste unsubstituiert oder durch einen oder mehrere Reste aus der Gruppe Halogen, CN, NO₂, C₁-C₄-Alkyl, C₁-C₄-Haloalkyl, C₁-C₄-Alkoxy und C₁-C₄-Haloalkoxy substituiert ist, bedeuten und
(Fall a) R³ in Formel (I) eine Rest aus der Gruppe der für R¹, R² und R⁴ möglichen Reste bedeutet oder
(Fall b) R³ in Formel (I) Chlor bedeutet.

3. Verfahren gemäß Anspruch 1 oder 2, **dadurch gekennzeichnet, daß** die Verbindung (I) 2,6-Dichlorbenzoxazol ist.

4. Verfahren gemäß einem der Ansprüche 1 bis 3, **dadurch gekennzeichnet, daß** die Reaktion in Gegenwart eines organischen oder anorganischen Lösungsmittels oder in Substanz durchgeführt wird.

5. Verfahren gemäß einem der Ansprüche 1 bis 4, **dadurch gekennzeichnet, daß** als Chlorierungsmittel Chlor, SO₂Cl₂, PCl₃, PCl₅, POCl₃, SCl₂, S₂Cl₂, SOCl₂ oder Gemische der genannten Mittel eingesetzt werden.

6. Verfahren gemäß einem der Ansprüche 1 bis 4, **dadurch gekennzeichnet, daß** als Chlorierungsmittel Chlor in Kombination mit PCl₃ oder PCl₅ eingesetzt wird.

7. Verfahren gemäß einem der Ansprüche 1 bis 6, **dadurch gekennzeichnet, daß** die Katalysatoren in einer Menge von 0,05 bis 10 Molprozent bezogen auf die eingesetzte Menge an Verbindung der Formel (II) eingesetzt werden.

8. Verfahren gemäß einem der Ansprüche 1 bis 7, **dadurch gekennzeichnet, daß** als Katalysatoren Montmorillonit oder Lewis Säuren eingesetzt werden.

9. Verfahren gemäß einem der Ansprüche 1 bis 8, **dadurch gekennzeichnet, daß** als Katalysatoren FeCl₃ oder AlCl₃ eingesetzt werden.

10. Verfahren gemäß einem der Ansprüche 1 bis 9, **dadurch gekennzeichnet, daß** die Reaktionstemperatur 20 bis 200°C beträgt.

## Claims

1. A process for preparing chlorobenzoxazoles of the formula (I), 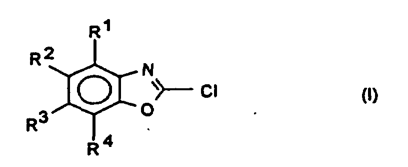 in which R¹, R² and R⁴ are each, independently of one another, H, halogen, CN, NO₂, C₁-C₅-alkyl, C₁-C₅-alkoxy, aryl or aryloxy, where each of the 4 lastmentioned radicals is unsubstituted or substituted, and
in case (a) R³ = H, halogen, CN, NO₂, C₁-C₅-alkyl, C₁-C₅-alkoxy, aryl or aryloxy, where each of the 4 lastmentioned radicals is unsubstituted or substituted, or
in case (b) R³ = chlorine,
which comprises reacting benzoxazoles of the formula (II), 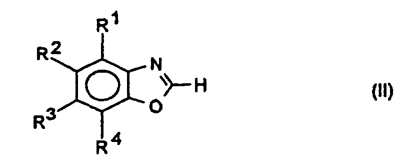 in which R¹, R² and R⁴ are as defined in formula (I) and R³ in case (a) is as defined in formula (I) and R³ in case (b) is hydrogen,
in the presence of an acidic catalyst with a chlorinating agent to give the monochlorination product (I) or in case (b) with an excess of the chlorinating agent to give the dichlorination product (I) in which R³ = chlorine.

2. The process as claimed in claim 1, wherein
R¹, R² and R⁴ in formula (I) are each, independently of one another, H, halogen, CN, NO₂, C₁-C₅-alkyl, C₁-C₅-haloalkyl, C₁-C₅-alkoxy, C₁-C₅-haloalkoxy, phenyl or phenoxy, where each of the 2 lastmentioned radicals is unsubstituted or substituted by one or more radicals selected from the group consisting of halogen, CN, NO₂, C₁-C₄-alkyl, C₁-C₄-haloalkyl, C₁-C₄-alkoxy and C₁-C₄-haloalkoxy, and
(Case a) R³ in formula (I) is a radical selected from the group of the radicals possible for R¹, R² and R⁴ or
(Case b) R³ in formula (I) is chlorine.

3. The process as claimed in claim 1 or 2, wherein the compound (I) is 2,6-dichlorobenzoxazole.

4. The process as claimed in any of claims 1 to 3, wherein the reaction is carried out in the presence of an organic or inorganic solvent or neat.

5. The process as claimed in any of claims 1 to 4, wherein the chlorinating agent used is chlorine, SO₂Cl₂, PCl₃, PCl₅, POCl₃, SCl₂, S₂Cl₂, SOCl₂ or a mixture of the abovementioned agents.

6. The process as claimed in any of claims 1 to 4, wherein the chlorinating agent used is chlorine in combination with PCl₃ or PCl₅.

7. The process as claimed in any of claims 1 to 6, wherein the catalysts are employed in an amount of from 0.05 to 10 mol percent, based on the amount of compound of the formula (II) used.

8. The process as claimed in any of claims 1 to 7, wherein the catalyst employed is montmorillonite or a Lewis acid.

9. The process as claimed in any of claims 1 to 8, wherein the catalyst employed is FeCl₃ or AlCl₃.

10. The process as claimed in any of claims 1 to 9, wherein the reaction temperature is from 20 to 200°C.

## Revendications

1. Procédé de préparation de chlorobenzoxazoles de formule (I) 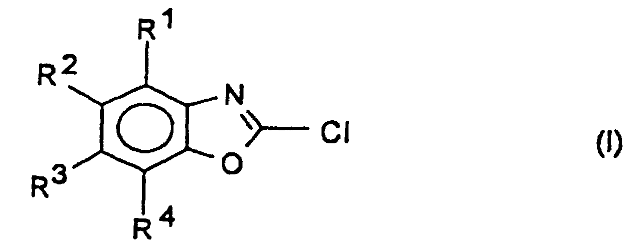 où R¹, R² et R⁴ indépendamment les uns des autres représentent chacun un atome d'hydrogène, un atome d'halogène, un groupe CN, NO₂, alkyle en C₁-C₅, alkoxy en C₁-C₅, aryle ou aryloxy, chacun des 4 restes précités peut être non substitué ou substitué, et
dans le cas (a) R³ = un atome d'hydrogène, un atome d'halogène, un groupe CN, NO₂, alkyle en C₁-C₅, alkoxy en C₁-C₅, aryle ou aryloxy, chacun des 4 restes précités peut être non substitué ou substitué, ou
dans le cas (b) R³ = chlore,
**caractérisé en ce que** qu'on fait réagir des benzoxazoles de formule (II) 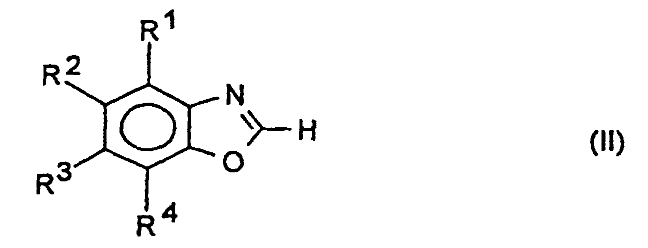 dans laquelle R¹, R² et R⁴ possèdent les mêmes significations qu'à la formule (I) et R³ dans le cas de (a) est défini comme à la formule (I) ou R³ dans le cas de (b) représente un atome d'hydrogène,
en présence d'un catalyseur acide sur un agent de chloration en obtenant le produit de monochloration (I), ou dans le cas de (b) sur un excès en agent de chloration en obtenant le produit de dichloration (I), où R³ représente un atome de chlore.

2. Procédé selon la revendication 1,
**caractérisé en ce que**
R¹, R² et R⁴ à la formule (I) indépendamment les uns des autres représentent chacun un atome d'hydrogène, un atome d'halogène, un groupe CN, NO₂, alkyle en C₁-C₅, haloalkyle en C₁-C₅, alkoxy en C₁-C₅, haloalkoxy en C₁-C₅, phényle ou phénoxy, chacun des deux derniers restes cités pouvant être substitué par un ou plusieurs restes pris dans le groupe comprenant un atome d'halogène, un groupe CN, NO₂, alkyle en C₁-C₄, haloalkyle en C₁-C₄, alkoxy en C₁-C₄ et haloalkoxy en C₁-C₄, et
(cas a) R³ à la formule (I) représente un reste pris dans le groupe des restes possibles pour R¹, R² et R⁴, ou
(cas b) R³ à la formule (I) représente chlore.

3. Procédé selon la revendication 1 ou 2, **caractérisé en ce que** le composé (I) est le 2,6-dichlorobenzoxazole.

4. Procédé selon l'une des revendications 1 à 3, **caractérisé en ce que** la réaction est mise en oeuvre en présence d'un solvant organique ou inorganique ou en masse.

5. Procédé selon l'une des revendications 1 à 4, **caractérisé en ce qu'**on utilise comme agent de chloration le chlore, SO₂Cl₂, PCl₃, PCl₅, POCl₃, SCl₂, S₂Cl₂, SOCl₂ ou des mélanges des agents précités.

6. Procédé selon l'une des revendications 1 à 4, **caractérisé en ce qu'**on utilise comme agent de chloration le chlore en combinaison avec PCl₃ ou PCl₅.

7. Procédé selon l'une des revendications 1 à 6, **caractérisé en ce qu'**on utilise les catalyseurs en une quantité de 0,05 à 10 % molaires par rapport à la quantité de composé de formule (II).

8. Procédé selon l'une des revendications 1 à 7, **caractérisé en ce qu'**on utilise comme catalyseurs la montmorillonite ou des acides de Lewis.

9. Procédé selon l'une des revendications 1 à 8, **caractérisé en ce qu'**on utilise comme catalyseurs FeCl₃ ou AlCl₃.

10. Procédé selon l'une des revendications 1 à 9, **caractérisé en ce que** la température réactionnelle est de 20 à 200°C.
